(19) **Europäisches Patentamt / European Patent Office / Office européen des brevets**

(11) **EP 3 424 466 B1**

(12) # EUROPEAN PATENT SPECIFICATION

(45) Date of publication and mention
of the grant of the patent:
**15.06.2022 Bulletin 2022/24**

(21) Application number: **17760003.8**

(22) Date of filing: **28.02.2017**

(51) International Patent Classification (IPC):
**A61F 2/28** (2006.01)    **A61L 27/00** (2006.01)
**A61L 27/06** (2006.01)    **A61L 27/30** (2006.01)
**A61L 27/50** (2006.01)    **A61L 27/54** (2006.01)
**A61L 31/02** (2006.01)    **A61L 31/08** (2006.01)
**A61L 31/14** (2006.01)    **A61L 31/16** (2006.01)

(52) Cooperative Patent Classification (CPC):
**A61F 2/28; A61L 27/06; A61L 27/306; A61L 27/50;
A61L 27/54; A61L 31/028; A61L 31/088;
A61L 31/14; A61L 31/16;** A61L 2300/102;
A61L 2300/404; A61L 2300/602

(86) International application number:
**PCT/JP2017/007813**

(87) International publication number:
**WO 2017/150537 (08.09.2017 Gazette 2017/36)**

(54) **ANTIBACTERIAL APPARATUS FOR IN-VIVO IMPLANTATION**

ANTIBAKTERIELLE VORRICHTUNG ZUR IN-VIVO-IMPLANTATION

APPAREIL ANTIBACTÉRIEN POUR IMPLANTATION IN VIVO

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.02.2016 JP 2016038139**

(43) Date of publication of application:
**09.01.2019 Bulletin 2019/02**

(73) Proprietor: **Medtronic Sofamor Danek, Co., Ltd.
Osaka-shi, Osaka 553-0003 (JP)**

(72) Inventors:
• **SUGINO, Atsushi
Osaka-shi
Osaka 553-0003 (JP)**
• **FUKUZAKI, Satoshi
Tsu-shi
Mie 514-8507 (JP)**

(74) Representative: **FRKelly
27 Clyde Road
Dublin D04 F838 (IE)**

(56) References cited:
WO-A1-2014/150904    WO-A2-2007/005842
WO-A2-2015/001366    JP-A- S 622 947
JP-A- 2002 536 048    JP-A- 2013 528 411
US-B1- 8 927 004

• KEUN-TAEK OH ET AL: "Electrochemical properties of suprastructures galvanically coupled to a titanium implant", JOURNAL OF BIOMEDICAL MATERIALS RESEARCH PART B: APPLIED BIOMATERIALS, vol. 70B, no. 2, 6 April 2004 (2004-04-06) , pages 1-27, XP055624741,
• FERRARIS S ET AL: "Antibacterial titanium surfaces for medical implants", MATERIALS SCIENCE AND ENGINEERING C, ELSEVIER SCIENCE S.A, CH, vol. 61, 31 December 2015 (2015-12-31), pages 965-978, XP029403060, ISSN: 0928-4931, DOI: 10.1016/J.MSEC.2015.12.062
• HIROAKI TAKADAMA et al.: "Jinko Kansetsuyo Titanium Kinzoku eno Seitai Kassei to Kokinsei no Fuyo", Bulletin of Institute of Life and Health Sciences, Chubu University = Annual report of Research Institute of Life and Health Sciences, vol. 7, March 2011 (2011-03), pages 61-66, XP009519694, ISSN: 1880-3040

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to an implant device, and specifically relates to an implant device that achieves an antimicrobial property while it is implanted in vivo for use.

BACKGROUND ART

A variety of instruments such as cages, wires, plates, screws, rods, connectors, crosslinks, hooks, set screws, artificial vertebral bodies, and artificial intervertebral discs are used as orthopedic implant devices. These instruments may be contaminated with microorganisms when they are implanted in vivo for use. If an implant device is colonized by microorganisms, it is very difficult to prevent microbial growth. To prevent microbial growth and infection in the body, it is necessary to perform surgery to remove the implant device for replacement with a new one, which places an enormous burden on the patient. It is desired that an antimicrobial implant device be provided.

As a technique for imparting an antimicrobial property to an implant device, a medical implant system has been proposed (Patent Literature 1) which comprises a metal component containing an antimicrobial metal disposed on an external surface of the implant body; a power source having a first terminal and a second terminal, one of the terminals being in electrical communication with the metal component; and an insulator placed in a current path between the first terminal of the power source and the second terminal of the power source, preventing current flowing from the first terminal from reaching the second terminal without completing a circuit including a conductive body tissue or fluid adjacent to the external surface of the implant system when implanted. Patent Literature 1 discloses that examples of the antimicrobial metal include silver, copper, both silver and copper, both silver and cadmium, and a combination of silver, copper, and cadmium. In Patent Literature 1, the external power source equipment and the terminals are required in addition to the implant body to achieve an antimicrobial property, and a treatment or an external operation is needed to impart an antimicrobial property. Further improvement is required from the viewpoint of reducing the burden on patients.

CITATION LIST

PATENT LITERATURE

PTL 1: Japanese Patent No. 5175185
US 8 927 004 B1 discloses an implant releasing antimicrobial metal ions comprising a first and second metal.

SUMMARY OF INVENTION

TECHNICAL PROBLEM

It is an object of the present invention to provide an implant device that can achieve an antimicrobial property, simply by being implanted in vivo, to reduce the burden on patients.

SOLUTION TO PROBLEM

The present invention is directed to an implantable device as defined in claim 1.

ADVANTAGEOUS EFFECTS OF INVENTION

The implant device of the present invention, once implanted in vivo, can achieve an antimicrobial property without requiring an external operation or treatment, thereby significantly reducing the burden on patients.

BRIEF DESCRIPTION OF DRAWINGS

Fig. 1 is an explanatory schematic diagram showing a state in which an antimicrobial implant device of the present invention is used.
Figs. 2(a), (b), and (c) are a top view, a side view, and a cross-sectional view, respectively, of an antimicrobial O-ring of a first embodiment.

Figs. 3(a), (b), and (c) are a top view, a side view, and a cross-sectional view, respectively, of an antimicrobial O-ring of a second embodiment.

Figs. 4(a), (b), and (c) are a top view, a side view, and a cross-sectional view, respectively, of an antimicrobial C-ring of the first embodiment.

Figs. 5(a), (b), and (c) are a top view, a side view, and a cross-sectional view, respectively, of an antimicrobial C-ring of the second embodiment.

DESCRIPTION OF EMBODIMENTS

The present invention will be described with reference to the attached drawings.

An antimicrobial implant device of the present invention is an implant device as defined in claim 1. An electric circuit is formed between the first material and the second material when the device is implanted in vivo for use; and ions of the antimicrobial metal are eluted from the first material under a presence of an electrolyte to impart an antimicrobial property to a surface and/or a surrounding area of the first material. The antimicrobial implant device of the present invention is made of at least two materials, and simply because these two materials are contacted in vivo, a short circuit occurs to form an electric circuit.

Preferably, the cobalt-based alloy comprises cobalt in an amount of 29 to 69 mass%. Still more preferably, the cobalt-based alloy is a cobalt-chromium alloy comprising chromium in an amount of 18 to 30 mass%.

Preferably, the titanium or the titanium-based alloy comprises titanium in an amount of 68 to 100 mass%.

More preferably, a ratio of a surface area of the second material to a surface area of the first material (second material/first material) = 0.2 to 10.8.

When the first material made of the cobalt-based alloy comprising cobalt in an amount of 29 to 69 mass% and the second material made of the titanium-based alloy comprising titanium in an amount of 68 to 100 mass% are used in combination, cobalt ions are eluted from the first material in an amount of 7 nmol/l to 81 $\mu$mol/l to provide a good antimicrobial property.

An antimicrobial implant device of the present invention will be described in detail with reference to the attached drawings.

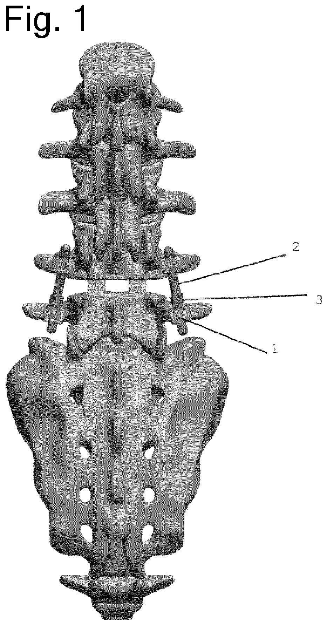

Fig. 1 is an explanatory schematic diagram showing a state in which the antimicrobial implant device (ring) of the present invention is used. Fig. 1 shows a rod 2 that is supported between two screws 1, each of which is implanted in a vertebra (pedicle), and an antimicrobial ring 3 that is fit on a circumference of the rod 2.

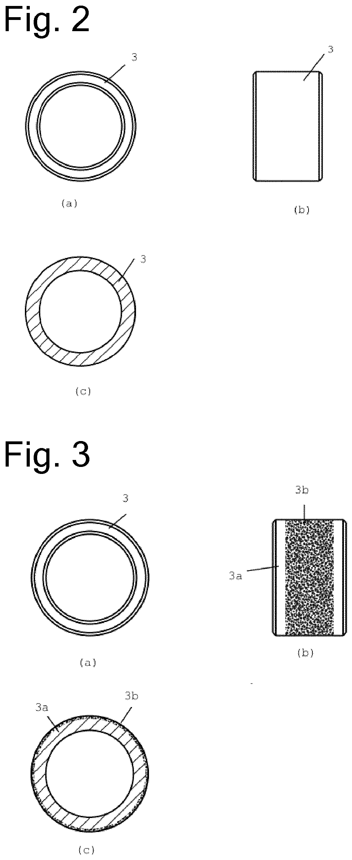

Figs. 2(a), (b), and (c) are a top view, a side view, and a cross-sectional view, respectively, of the antimicrobial O-ring 3 of a first embodiment. The antimicrobial O-ring 3 is fit around the rod 2 for use. The antimicrobial O-ring 3 shown in Fig. 2 is made entirely of a first material.

Figs. 3(a), (b), and (c) are a top view, a side view, and a cross-sectional view, respectively, of an antimicrobial O-ring 3 of a second embodiment. The antimicrobial O-ring 3 shown in Fig. 3 includes a solid region 3a made of the first material and a porous region 3b made of the first material or a second material. The porous region 3b is a region composed of intertwined or agglutinated fibers comprising the first material or the second material, or a region containing a sintered body of powder particles of the first material or the second material. The porous region 3b is laminated to coat the surface of the solid region 3a. To laminate the porous region and the solid region, compression molding, sintering, diffusion bonding, additive manufacturing using an electron beam or laser, metal injection molding, spark plasma sintering, or a combination of these methods can be suitably used. When the porous region 3b is made of the second material, the surface area of the second material is increased to increase the ratio of the surface area of the second material to the surface area of the first material. As shown in the results of the examples described below, the antimicrobial property improves as the ratio of the surface area of the second material increases.

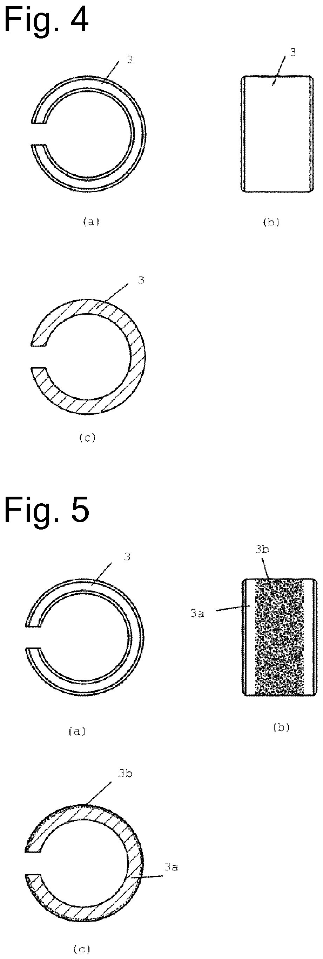

Figs. 4(a), (b), and (c) are a top view, a side view, and a cross-sectional view, respectively, of an antimicrobial C-ring 3 of the first embodiment. The antimicrobial C-ring 3 is fit around the rod 2 for use. The antimicrobial C-ring 3 shown in Fig. 4 is made entirely of the first material.

Figs. 5(a), (b), and (c) are a top view, a side view, and a cross-sectional view, respectively, of an antimicrobial C-ring 3 of the second embodiment. As in the antimicrobial O-ring shown in Fig. 3, the antimicrobial C-ring 3 shown in Fig. 5 includes a solid region 3a made of the first material and a porous region 3b made of the first material or the second material, wherein the porous region 3b is laminated to coat the surface of the solid region 3a.

EXAMPLES

The present invention will be hereinafter described in detail with examples; however, the invention is not limited to these examples.

[Example 1]

(Preparation of Samples)

A commercially available biocompatible titanium alloy (titanium content: 88 to 91 mass%) and a commercially available biocompatible cobalt-chromium alloy (cobalt content: 58 to 69 mass%, chromium content: 26 to 30 mass%) were processed into 3-mm-thick coin-shaped samples (only the titanium alloy of Sample No. 3 was processed into a flat plate-shaped sample) having the various areas shown in Table 1, and these samples were laminated in the various combinations shown in Table 1 and shorted to each other. As comparative samples, a 1-mm-thick flat plate-shaped polyethylene sample (control), a 3-mm-thick coin-shaped sample made of the cobalt-chromium alloy (Comparison 1), and a 3-mm-thick coin-shaped sample made of the titanium alloy (Comparison 2) were similarly prepared.

[Table 1]

| Table 1 Sample Conditions | | | |
|---|---|---|---|
| | | Surface Area/mm$^2$ | |
| Sample No. | Area Ratio* | Titanium Alloy | Cobalt-Chromium Alloy |
| 1 | 1.0 | 518 | 518 |
| 2 | 3.3 | 1696 | 518 |
| 3 | 10.8 | 5600 | 518 |
| 4 | 0.2 | 103 | 518 |
| Comparison 1 | - | - | 518 |
| Comparison 2 | - | 518 | - |
| Control | - | Polyethylene | |
| * Area Ratio = Surface Area of Titanium Alloy/Surface Area of Cobalt-Chromium Alloy | | | |

(Antimicrobial Property Test)

An antimicrobial property was evaluated with reference to JIS Z 2801: Antibacterial products - Test for antibacterial activity and efficacy.

Each of the samples shown in Table 1 was placed in a petri dish, and 40 μl of *Staphylococcus aureus* (NBRC 12732) suspended in a nutrient broth (1/100) was added dropwise. A polyethylene film was placed over the test bacterial suspension added. The suspension was cultured at 35°C for 24 hours, and then cells were washed out from the sample. The resulting wash-out suspension was serially diluted in a 10-fold dilution series and cultured at 35°C for 24 hours using the agar plate culture method, and the number of viable cells was calculated from the number of colony forming units (CFUs) formed. The difference between the logarithmic value of the number of viable cells on the polyethylene sample as a control after 24-hour contact (Nc) and the logarithmic value of the number of viable cells on each sample (N) was determined as the antimicrobial activity value.

[Math. 1]

$$\text{Antimicrobial activity value} = \log(Nc/N)$$

Table 2 shows the number of viable cells and the antimicrobial activity value for each sample. A sample having an antimicrobial activity value of 2.0 or more was determined to have antimicrobial efficacy. The number of viable cells decreased (the antimicrobial activity value increased) as the area ratio (the surface area of the sample made of the titanium alloy/the surface area of the sample made of the cobalt-chromium alloy) increased.

[Table 2]

| Table 2 Results of Antimicrobial Property Test | | |
|---|---|---|
| Sample | Number of Viable Cells/CFU/Coupon | Antimicrobial Activity Value |
| Control: Polyethylene | $1.1 \times 10^6$ | - |
| 1 | $6.8 \times 10^3$ | 2.21 |
| 2 | $2.4 \times 10^3$ | 2.66 |
| 3 | $4.0 \times 10^2$ | 3.44 |
| Comparison 1 | $9.2 \times 10^3$ | 2.08 |

[Example 2]

(Preparation of Samples)

An implant device made of the titanium alloy and the cobalt-chromium alloy (the area ratio of the titanium alloy to the cobalt-chromium alloy was set to 0.89) was prepared (implant device 1). As a comparative example, an implant device made of only the titanium alloy having completely the same shape was similarly prepared (implant device 2).

[Table 3]

| Table 3 Composition of Implant Device | | Combination of Materials | |
|---|---|---|---|
| Implant Device No. | Area Ratio* | Titanium Alloy | Cobalt-Chromium Alloy |
| 1 | 0.89 | O | O |
| 2 | --- | O | |
| * Area Ratio = Surface Area of Member Made of Titanium Alloy/Surface Area of Member Made of Cobalt-Chromium Alloy | | | |

(Antimicrobial Property Test)

Each of the implant devices was placed in a polypropylene tube, and immersed in 1 ml of *Staphylococcus aureus* (NBRC 12732) suspended in a nutrient broth (1/100). After 24 hours of culture at 35°C, the culture medium was collected. The collected culture medium was serially diluted in a 10-fold dilution series and cultured at 35°C for 24 hours using the agar plate culture method, and the number of viable cells was determined from the number of colony forming units (CFUs) formed.

Table 4 shows the number of viable cells and the antimicrobial activity value for each implant device. In the implant device 1 in which the area ratio of the titanium alloy to the cobalt-chromium alloy was appropriately set, the number of viable cells significantly decreased. In contrast, in the implant device 2 made of the titanium alloy only, a decrease in the number of viable cells was not observed.

[Table 4]

| Table 4 Results of Antimicrobial Property Test for Implant Device | | |
|---|---|---|
| Implant Device No. | Number of Viable Cells/CFU/Coupon | Antimicrobial Activity Value |
| Polyethylene | $7.6 \times 10^7$ | - |
| 1 | $1.2 \times 10^5$ | 2.80 |
| 2 | $7.0 \times 10^7$ | 0.04 |

## Claims

1.   An antimicrobial implant device consisting of a rod comprising a second material and a ring comprising a first material that is fitted on at least a part of a circumference of the rod, wherein

at least a surface of the first material comprises an antimicrobial metal, wherein the antimicrobial metal is a cobalt-based alloy;
wherein the second material is titanium or a titanium-based alloy;
at least a surface of the second material is nobler than the first material;
an electric circuit is formed between the first material and the second material when the device is implanted in vivo for use;
ions of the antimicrobial metal are eluted from the first material under a presence of an electrolyte to impart an antimicrobial property to a surface and/or a surrounding area of the first material.

2.  The antimicrobial implant device according to claim 1, wherein the cobalt-based alloy comprises cobalt in an amount of 29 to 69 mass%.

3.  The antimicrobial implant device according to claim 1 or 2, wherein the cobalt-based alloy is a cobalt-chromium alloy.

4.  The antimicrobial implant device according to claim 3, wherein the cobalt-chromium alloy comprises chromium in an amount of 18 to 30 mass%.

5.  The antimicrobial implant device according to any one of claims 1 to 4, wherein the titanium or the titanium-based alloy comprises titanium in an amount of 68 to 100 mass%.

6.  The antimicrobial implant device according to any one of claims 1 to 5 comprising a porous region composed of intertwined or agglutinated fibers comprising the first material and/or a porous region composed of intertwined or agglutinated fibers comprising the second material.

7.  The antimicrobial implant device according to claim 6 comprising a porous structure composed of intertwined or agglutinated fibers comprising the first material and/or a porous structure composed of intertwined or agglutinated fibers comprising the second material.

8.  The antimicrobial implant device according to any one of claims 1 to 5 comprising a sintered portion of powder particles of the first material or the second material.

9.  The antimicrobial implant device according to claim 8 having a sintered body of powder particles of the first material or the second material.

10.  The antimicrobial implant device according to any one of claims 1 to 9 wherein a ratio of a surface area of the second material to a surface area of the first material (second material/first material) is in a range of 0.2 to 10.8.

**Patentansprüche**

1.  Antimikrobielle Implantatvorrichtung, die aus einem Stab, der ein zweites Material umfasst, und einem Ring, der ein erstes Material umfasst, besteht, der auf wenigstens einen Teil eines Umfangs des Stabes montiert ist, wobei

wenigstens eine Oberfläche des ersten Materials ein antimikrobielles Metall umfasst, wobei das antimikrobielle Metall eine Legierung auf Kobaltbasis ist;
wobei das zweite Material Titan oder eine Legierung auf Titanbasis ist;
wenigstens eine Oberfläche des zweiten Materials edler als das erste Material ist;
ein elektrischer Stromkreis zwischen dem ersten Material und dem zweiten Material ausgebildet ist, wenn die Vorrichtung zur Verwendung in vivo implantiert ist;
Ionen des antimikrobiellen Metalls aus dem ersten Material unter einer Anwesenheit eines Elektrolyten eluiert werden, um einer Oberfläche und/oder einem Umgebungsbereich des ersten Materials eine antimikrobielle Eigenschaft zu verleihen.

2.  Antimikrobielle Implantatvorrichtung nach Anspruch 1, wobei die Legierung auf Kobaltbasis Kobalt in einer Menge von 29 bis 69 Massen-% umfasst.

3. Antimikrobielle Implantatvorrichtung nach Anspruch 1 oder 2, wobei die Legierung auf Kobaltbasis eine Kobalt-Chrom-Legierung ist.

4. Antimikrobielle Implantatvorrichtung nach Anspruch 3, wobei die Kobalt-Chrom-Legierung Chrom in einer Menge von 18 bis 30 Massen-% umfasst.

5. Antimikrobielle Implantatvorrichtung nach einem der Ansprüche 1 bis 4, wobei das Titan oder die Legierung auf Titanbasis Titan in einer Menge von 68 bis 100 Massen-% umfasst.

6. Antimikrobielle Implantatvorrichtung nach einem der Ansprüche 1 bis 5, die eine poröse Region, die aus verflochtenen oder agglutinierten Fasern besteht, die das erste Material umfassen, und/oder eine poröse Region umfasst, die aus verflochtenen oder agglutinierten Fasern besteht, die das zweite Material umfassen.

7. Antimikrobielle Implantatvorrichtung nach Anspruch 6, die eine poröse Struktur, die aus verflochtenen oder agglutinierten Fasern besteht, die das erste Material umfassen, und/oder eine poröse Struktur umfasst, die aus verflochtenen oder agglutinierten Fasern besteht, die das zweite Material umfassen.

8. Antimikrobielle Implantatvorrichtung nach einem der Ansprüche 1 bis 5, die einen gesinterten Abschnitt von Pulverpartikeln des ersten Materials oder des zweiten Materials umfasst.

9. Antimikrobielle Implantatvorrichtung nach Anspruch 8, die einen gesinterten Körper von Pulverpartikeln des ersten Materials oder des zweiten Materials aufweist.

10. Antimikrobielle Implantatvorrichtung nach einem der Ansprüche 1 bis 9, wobei ein Verhältnis eines Oberflächenbereichs des zweiten Materials zu einem Oberflächenbereich des ersten Materials (zweites Material / erstes Material) in einem Bereich von 0,2 bis 10,8 liegt.

## Revendications

1. Dispositif d'implant antimicrobien constitué d'une tige comprenant un second matériau et d'un anneau comprenant un premier matériau qui est ajusté sur au moins une partie d'une circonférence de la tige, dans lequel

   au moins une surface du premier matériau comprend un métal antimicrobien, le métal antimicrobien étant un alliage à base de cobalt ;
   le second matériau étant du titane ou un alliage à base de titane ;
   le second matériau étant plus noble que le premier matériau ;
   un circuit électrique étant formé entre le premier matériau et le second matériau lorsque le dispoisitif est implanté in vivo pour son utilisation ;
   des ions du métal antimicrobien étant élués à partir du premier matériau en présence d'un électrolyte pour conférer une propriété antimicrobienne à une surface et/ou à une zone environnante du premier matériau.

2. Dispositif d'implant antimicrobien selon la revendication 1, l'alliage à base de cobalt comprenant du cobalt en une quantité de 29 à 69 % en masse.

3. Dispositif d'implant antimicrobien selon la revendication 1 ou 2, l'alliage à base de cobalt étant un alliage cobalt-chrome.

4. Dispositif d'implant antimicrobien selon la revendication 3, l'alliage cobalt-chrome comprenant du chrome en une quantité de 18 à 30 % en masse.

5. Dispositif d'implant antimicrobien selon l'une quelconque des revendications 1 à 4, le titane ou l'alliage à base de titane comprenant du titane en une quantité de 68 à 100 % en masse.

6. Dispositif d'implant antimicrobien selon l'une quelconque des revendications 1 à 5, comprenant une région poreuse composée de fibres entrelacées ou agglutinées comprenant le premier matériau et/ou une région poreuse composée de fibres entrelacées ou agglutinées comprenant le second matériau.

**7.** Dispositif d'implant antimicrobien selon la revendication 6, comprenant une structure poreuse composée de fibres entrelacées ou agglutinées comprenant le premier matériau et/ou une structure poreuse composée de fibres entrelacées ou agglutinées comprenant le second matériau.

**8.** Dispositif d'implant antimicrobien selon l'une quelconque des revendications 1 à 5, comprenant une partie frittée de particules de poudre du premier matériau ou du second matériau.

**9.** Dispositif d'implant antimicrobien selon la revendication 8, ayant un corps fritté de particules de poudre du premier matériau ou du second matériau.

**10.** Dispositif d'implant antimicrobien selon l'une quelconque des revendications 1 à 9, un rapport d'une superficie du second matériau à une superficie du premier matériau (second matériau/premier matériau) étant compris entre 0,2 et 10,8.

# Fig. 1

# Fig. 2

(a)

(b)

(c)

# Fig. 3

(a)

3b

3a

(b)

3a    3b

(c)

# Fig. 4

(a)

(b)

(c)

# Fig. 5

(a)

3b

3a

(b)

3b

3a

(c)

**EP 3 424 466 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5175185 B
- US 8927004 B1